# EUROPEAN PATENT SPECIFICATION

(11) **EP 1 001 758 B1**
(45) Date of publication and mention of the grant of the patent: **20.11.2002**
(21) Application number: 98930032.2
(22) Date of filing: 24.06.1998
(51) Int. Cl.: A61K 31/195, C12N 15/09, C12N 15/12, C12Q 1/68, C12N 5/10, G01N 33/569, A61P 3/00, A61P 3/04, A61P 3/10

(54) **METHOD OF SCREENING OF POTENTIAL DRUGS AGAINST OBESITY, CAPABLE OF MODULATING THE REGULATION OF UCP-2**
AUSWAHLMETHODE FÜR POTENTIELLE MITTEL GEGEN FETTLEIBIGKEIT, ZUR REGULIERUNG DES UCP-2
PROCEDE DE TRI DE MEDICAMENTS POTENTIELS CONTRE L'OBESITE PERMETTANT DE MODULER LA REGULATION DE L'UCP-2

(30) Priority: 26.06.1997 SE 9702457
(43) Date of publication of application: 24.05.2000
(73) Proprietor: BIOVITRUM AB, 112 76 Stockholm (SE)
(72) Inventor: LIND, Peter, S-752 29 Uppsala (SE); WALUM, Erik, S-184 61 kersberga (SE)
(74) Representative: Höglund, Lars
(86) International application number: SE9801232
(87) International publication number: WO99000123

(56) References cited:
- EP-A- 0 148 686
- WO-A-96/16031
- WO-A-96/40226
- WO-A-98/10059
- WO-A-98/31396
- FR-A- 2 733 513
- US-A- 4 623 619
- BIOCHEMICAL AND BIOPHYSICAL RESEARCH COMMUNICATIONS, Volume 237, 1997, MICHIO SHIMABUKURO et al., "Induction of Uncoupling Protein-2 mRNA by Troglitazone in the Pancreatic Islets of Zucker Diabetic Fatty Rats", pages 359-361.
- STN INTERNATIONAL, File CA, CHEMICAL ABSTRACTS, Volume 127, No. 16, 20 October 1997, (Columbus, Ohio, US), OKUNO AKIRA et al., "Effects of Thiazolidines on Adipose Tissue of Zucker Fatty Rat", Abstract No. 214966; & DIABETES FRONT. (1997), 8(4), 499-501.
- BIOCHEMICAL PHARMACOLOGY, Volume 54, 1997, MASOUD GHORBANI et al., "Hypertrophy of Brown Adipocytes in Brown and White Adipose Tissues and Reversal of Diet-Induced Obesity in Rats Treated with a Beta3-Adrenoceptor Agonist", pages 121-131.
- INTERNATIONAL JOURNAL OF OBESITY, Volume 21, 1997, M. GHORBANI et al., "Appearance of Brown Adipocytes in White Adipose Tissue During CL 316,243-Induced Reversal of Obesity and Diabetes in Zucker fa/fa Rats", pages 465-475.
- J. CLIN. INVEST., Volume 97, No. 12, June 1996, ITSURO NAGASE et al., "Expression of Uncoupling Protein in Skeletal Muscle and White Fat of Obese Mice Treated with Thermogenic beta3-Adrenergic Agonist", pages 2898-2904.
- STN INTERNATIONAL, File CAPLUS, CAPLUS Accession No. 1997:145811, FLEURY CHRISTOPHE et al., "Uncoupling Protein - 2 : a Novel Gene Linked to Obesity and Hyperinsulinemia"; & NAT. GENET., (1997), 15(3), 269-272.
- STN INTERNATIONAL, File CAPLUS, CAPLUS Accession No. 1998:531544, KUBOTA TAKANORI et al., "Molecular Screening of Uncoupling Protein 2 Gene in Patients with Noninsulin-Dependent Diabetes Mellitus or Obesity"; & J. CLIN. ENDOCRINOL. METAB., (1998), 83(8), 2800-2804.

## Description

### Introduction

The present invention relates to a method for screening for potential drugs against obesity, metabolic syndrome and/or non-insulin dependent diabetes mellitus comprising the measurement of UCP-2 activity by biochemical, chemical or physical methods.

### Background.

Obesity is a disease with strongly increasing prevalence, and has reached epidemic proportions in the industrialized world. This disease is essentially characterized by an unbalance between energy intake and expenditure, which, without interference, leads to an ever increase in adipose tissue mass and body weight.

Appetite and energy intake is influenced by several hormonal effectors and neurotransmitters acting in the peripheral as well as the central nervous system. Examples of neurotransmitters acting to increase appetite and concomitantly body weight, are neuropeptide Y, melanin concentrating hormone, and galanin, as well as glucocorticoid hormones. Examples of hormones or neurotransmitters that counteract feeding and stimulate reduction in adipose mass are melanocortin, corticotropin releasing factor, as well as the recently described peptide hormone leptin.

Brown adipose tissue (BAT) is a well characterized tissue which is well developed in newborn mammals, including man. One important task of BAT is to generate heat and maintain body temperature homeostasis in newborns, as well as in small animals, e.g. rodents.

The uncoupling protein, UCP-1, occurs in mitochondria, and seems to be the most important protein for generating heat in BAT. It does so by burning calories using a pathway that allows dissipation of the proton electrochemical gradient across the inner mitochondrial membrane in BAT during fuel oxidation. The fuel oxidation process is uncoupled for oxidative phosphorylation of ADP to ATP, thus generating heat which is distributed from BAT to the rest of the body via the circulation. The physiological external stimulus for uncoupling activity in BAT is cold temperature. This will increase the sympathetic nervous system activity and release of catecholamines leading to stimulation of beta3 adrenoreceptors present on the surface of brown adipocytes.

Recently, a new protein denoted UCP-2 has been discovered, which is expressed not only in BAT, but also in white adipose tissue (WAT), skeletal muscle, lung, heart, placenta, etc. (Fleury C, et al. (1997) "Uncoupling protein-2: a novel gene linked to obesity and hyperinsulinemia" Nat Genet 15(3), 269-272; Gimeno, RE., et al., (1997) "Cloning and characterization of an uncoupling protein homolog: a potential molecular mediator of human thermogenesis" Diabetes 46(5), 900-906). The UCP-2 protein has a 59% identity to UCP-1, and is upregulated in WAT in mice in response to feeding. This is in contrast to UCP-1, which is physiologically upregulated by cold in mice.

WO 9616031, The Upjohn Company, discloses aminoguanidine carboxylates, e.g. [1-(hydrazinoiminomethyl)hydrazino]acetic acid for the treatment of non-insulin dependent diabetes mellitus. The novel and claimed compounds reduce the abnormally elevated blood glucose level and have an increased glucose tolerance.

### The invention

We have now found that a drug-induced upregulation of UCP-2 mRNA is possible. Furthermore, we have found that, as a consequence of this, the level of UCP-2 protein increases and mitochondrial activity and heat flow increase. This serves as a foundation of the invention related to the drug-induced increase of metabolic efficiency, increase in energy expenditure, and increase thermogenesis by genetic or transcriptional upregulation of UCP-2 in adipose tissue. Drugs that increase energy expenditure are useful in the treatment of obesity, non-insulin dependent diabetes, as well as the metabolic syndrome. Obesity can be caused by different reasons such as non-insulin dependent diabetes, increase in triglycerides, increase in carbohydrate bound energy and low energy expenditure.
An increase in energy expenditure includes the elevated utilization of both circulating and intracellular glucose and triglycerides, free or stored as glycogen or lipids as fuel for energy and/or heat production.
Our invention relates to a method for identifying a drug capable of modulating the regulation of UCP-2 mRNA, thus an increase of metabolic efficiency, increase in energy expenditure, and increase thermogenesis by genetic or transcriptional upregulation of UCP-2 in adipose tissue. The drug capable of modulating the regulation of UCP-2 mRNA is useful for treatment of obesity, metabolic syndrome and/or non-insulin dependent diabetes mellitus.
The invention is thus related to methods for screening for potential drugs against obesity, metabolic syndrome and/or non-insulin dependent diabetes mellitus as defined in the claims.
The measurement of UCP-2 activity as upregulation of UCP-2 transcription /mRNA can be done by biochemical, chemical or physical methods, all well known for persons skilled in the art.

Screening with pharmacological or biochemical methods can e.g. be performed on mice by the use of candidate drugs or on cell-lines such as 3T3-L1 or 3T3-F442A, that optionally can be differentiated to adipocyte like cells.
The gene for UCP-2 and a reporter-gene (e.g. E coli β-galactosidase, chloramphenicol acetyltransferase, alkaline phosphatase or firefly luciferase) can also be used for the measurement of the UCP-2 activity.

The invention is illustrated with four examples, using an aminoguanidine carboxyloic acid, [1-(hydrazinoiminomethyl)hydrazino]acetic acid, (AG) as the substance capable of modulating the regulation of UCP-2. This is, however, no limitation of the invention in its broadest aspects.

Figures
- Figure 1.: Upper panel: RNA blotting filter hybridized to ³²P-labelled human uncoupling protein-2 cDNA probe.
Bottom panel:RNA blotting filter hybridized to ³²P-labelled human β-actin cDNA probe.
- Figure 2.: Regulation of UCP-2 mRNA levels in white adipose and skeletal muscle tissue by AG.
- Figure 3: Effects of AG on mitochondrial activity in neuroblastoma cells.
- Figure 4: Effects of AG on reactive oxygen species in neuroblastoma cells
- Figure 5: Effects of AG on neuroblastoma cells
- Figure 6: Effects of AG on the level of UCP-protein in neuroblastoma cells

### Definitions

- C5BL/6J ob/ob mice: Obese mice homozygous for a point mutation in the *OB* gene
- SSPE: Sodium chloride (0.15 M), Sodium phosphate (10 mM), EDTA (1 mM), pH 7.4
- SDS: Sodium dodecylsulphate
- AG: [1-(hydrazinoiminomethyl)hydrazino]acetic acid

### Example 1. Upregulation of UCP-2

Six C57BL/6J ob/ob mice (Bomholtsgård, Denmark) were treated by peritoneal injection with either the compound AG (80 mg/kg) or saline (3 mice in each group). The mice were sacrified after 20 hours and intra-abdominal fat, and skeletal muscle samples were removed from each mouse. Total RNA was extracted from these samples using the guanidinium thiocyanate method essentially as described by Sambrook *et al*, (1989) "Molecular cloning: a laboratory manual", Cold Spring Harbor Laboratory Press, (2nd edition).
The tissues were homogenized, together with 6 ml GTC (4M guanidinium thiocyanate, 25 mM Na-citrate. 8.06 mM 2-mercaptoethanol, final adjusted pH 7.0) in a Polytron homogenizer (Brinkmann) at high speed for 1-2 minutes. 300 ml, 10% sodium lauryl sarcosinate was added to a final concentration of 0.5%, then mixed thoroughly. This was centrifuged at 5000g, 10 minutes at room temperature.
The supernatant was transfered to a 50 ml Falcon tube and drawn six times through a 22-gauge needle. The tissue/GTC solution was layered on a 5.7 M cesium chloride solution (buffered by 8 mM sodium acetate and sterile filtered) and centrifuged at 32000 rpm in a swing-out SW4 1 rotor for 17 hours at room temperature.
The pellets were dissolved in 2x200 ml diethylpyrocarbonate (DEPC) treated water and transfered to microcentrifuge tubes. One tenth of the volume of 3M sodium acetate and 2 volumes of 95% ethanol was then added, followed by precipitating the RNA in -20°C for more than 30 minutes. The RNA was collected by centrifugation at 15000g, 15 minutes at 4°C. The pellet was washed in 70% ethanol and centrifuged for 5 minutes at 15000g. The supernatant was removed and the pellet was vacuum dried for 5 minutes. The RNA was resuspended in 100ml DEPC-treated water and kept on ice.
The integrity of the RNA was confirmed by separation on a 1% MP agarose (Boehringer Mannheim) gel. The RNA was then capillary blotted onto a GeneScreen Plus (DuPont NEN Research Products) membrane. The protocols used for the transfer and detection of the RNA are found in the technical manual: "GeneScreen and GeneScreen Plus; Hybridization Transfer_{~}Membranes; Transfer and Detection Protocols", DuPont. NEN Research Products, 549 Albany St., Boston, MA, USA.

The cDNA probe used for detecting UCP-2 was derived from the I.M.A.G.E. consortium clone No. 440295 and was amplified from the pT7T3D-Pac vector (Pharmacia&Upjohn) by PCR using the two primers 5'-CCAGTCACGACGTTGTAAA-3' and 5'-CACAGGAAACAGCTATGAC-3'. The probe was purified from a low-melting agarose gel prior to ³²p labelling which was carried out with the RediPrime DNA labelling kit kit (Amersham).

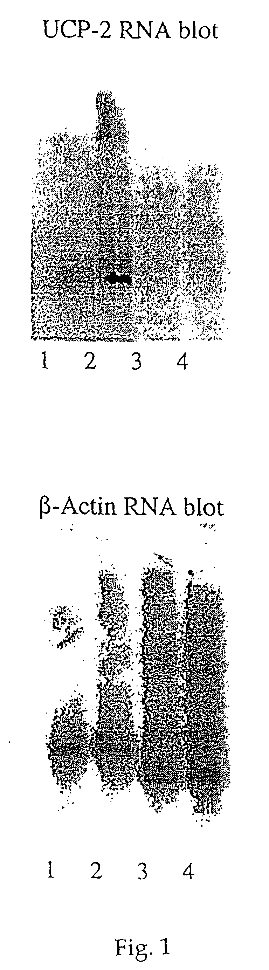

The RNA blotting filter was hybridized with the labelled probe in a 50% formamide solution at 42°C overnight with subsequent high stringency washing using four 2xSSPE for 15 minutes at room temperature, followed by two 2xSSPE, 2% SDS for 45 minutes at 65°C and, finally, two 15 minute washes in 0.1xSSPE at room temperature. After the final wash, filter bound radioactivity was detected and quantified using a Phosporlmager (Molecular Dynamics, Inc.). The results are shown in Figure 1. (top panel). After washing to remove filter-bound radioactivity, the RNA blotting filter was also hybridized to a ³²P-labelled probe based on human β-actin cDNA to serve as a control for the mRNA content of each lane. The results are shown in Figure 1. (bottom panel).

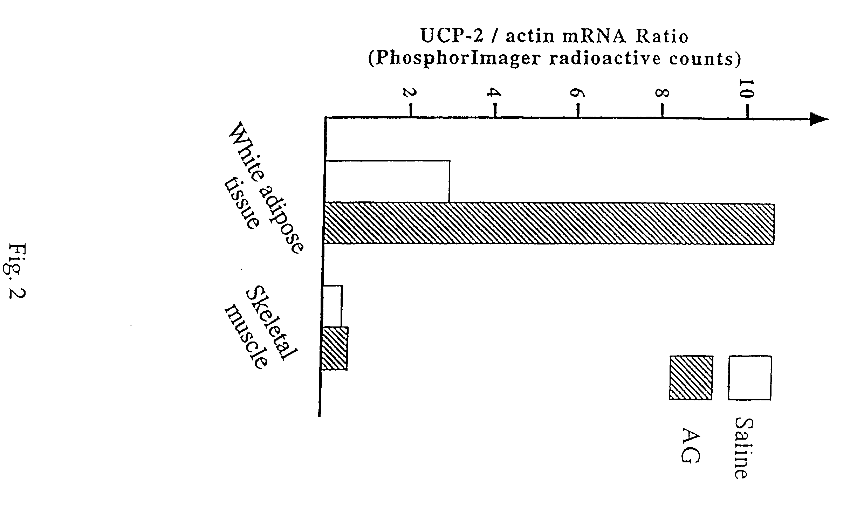

The relative expression levels of UCP-2 mRNA between samples of saline and AG treated mice, was calculated using the Phosphorlmager radioactive counts detected within the bands corresponding to UCP-2 mRNA, and normalized against the radioactive counts within the bands corresponding to actin, the latter of which do not change significantly between saline and AG treated samples. The data (illustrated in Figure 2.) indicate a 3.6-fold induction of UCP-2 mRNA in white adipose tissue after treatment with AG, and a 1.3 fold induction in skeletal muscle.

### Figure legends

Figure 1.Top panel: RNA blotting filter hybridized to ³²P-labelled human uncoupling protein-2 cDNA probe. Lane 1, white adipose tissue from saline treated mice; lane 2, white adipose tissue from AG treated mice; lane 4, skeletal muscle from saline treated mice; lane 4, skeletal muscle from AG treated mice.
Bottom panel: RNA blotting filter hybridized to ³²P-labelled human β-actin cDNA probe. Lane 1, white adipose tissue from saline treated mice; lane 2, white adipose tissue from AG treated mice; lane 4, skeletal muscle from saline treated mice; lane 4, skeletal muscle from AG treated mice.

Figure 2. Regulation of UCP-2 mRNA levels in white adipose and skeletal muscle tissue by AG. The white bars indicate saline treated control tissue, and the stippled bars indicate AG treated tissue. The UCP-2 mRNA levels were determined with a Phoshorlmager (Molecular Dynamics) and are normalized against actin mRNA levels.

From these figures it is clearly seen that UCP-2 mRNA is strongly upregulated by AG in white adipose tissue compared to treatment with placebo (saline). This is in contrast to skeletal muscle where only a marginal change in UCP-2 mRNA levels could be seen.

### Example 2. Increase in mitochondrial activity

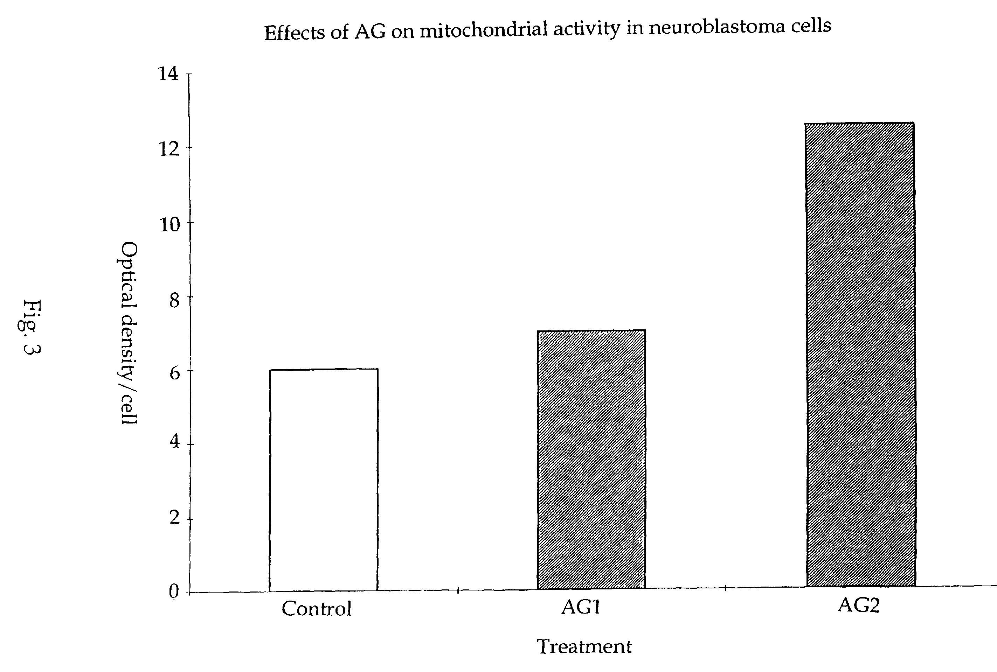

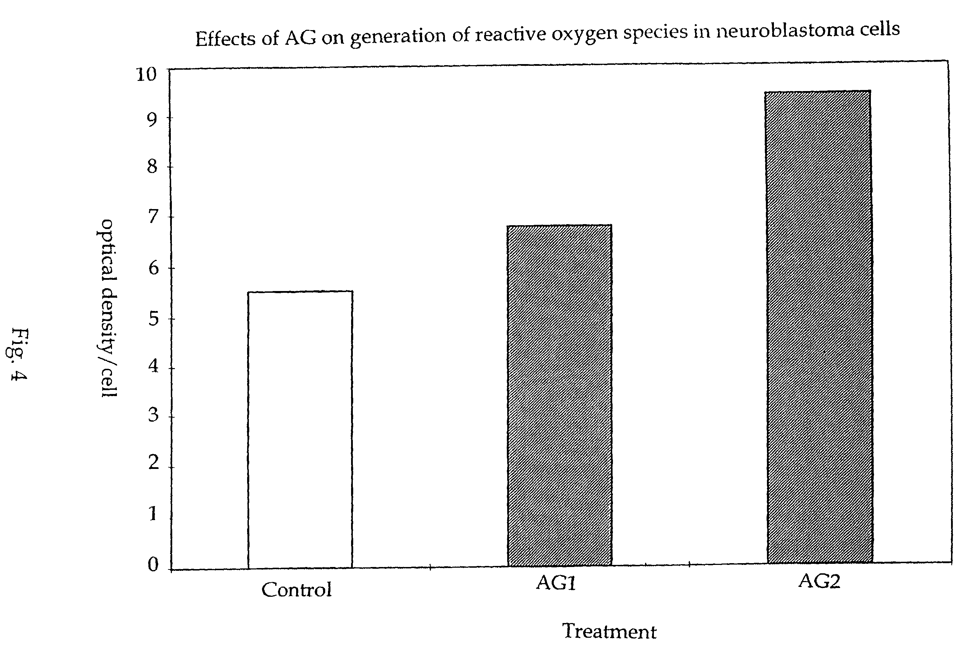

Further experiments have shown that aminoguanidine carboxylic acid (AG) increase mitochondrial activity (Figure 3) and the generation of reactive oxygen species (ROS) (Figure 4) in human neuroblasloma cells treated for 2 days.

### Example 3. Microcalorimetry.

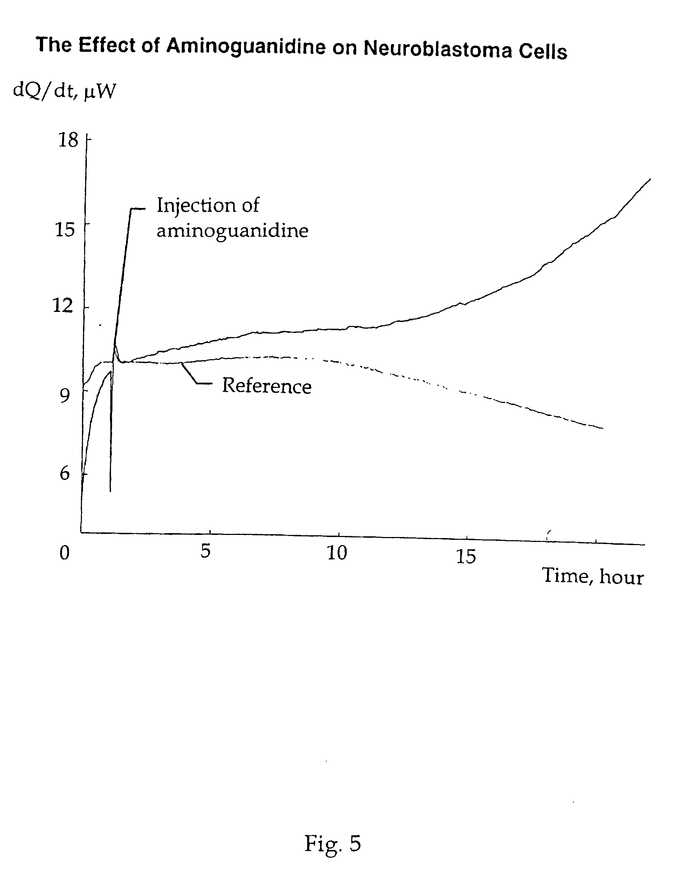

In SH-SY5Y cells, as well as in L6 (rat myocytes) cells AG has been shown, by microcalorimetry, to be thermogenic. The increase in heat production initiated by the AG required several hours of incubation to establish (Figure 5).

### Example 4. Cytometer

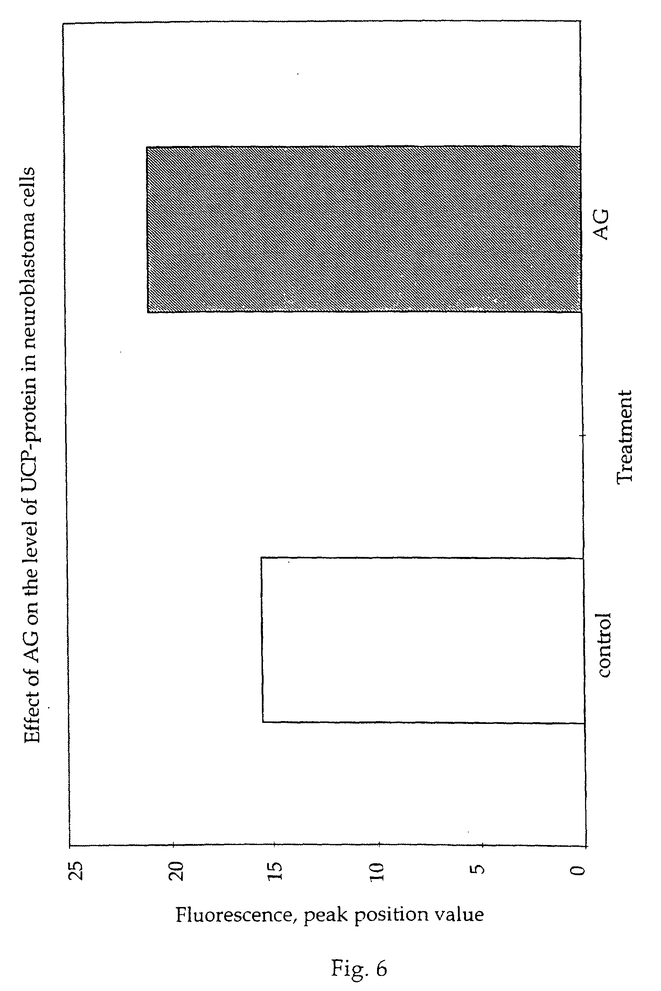

L6 cells and SH-SY5Y cells were shown to express UCP2 on the mRNA-level as well as the protein level. Using a laser scanner cytometer it was possible to show a sift in the peak fluorescence of antibody labelled UCP2 protein in SH-SY5Y cells after AG treatment. Increases in the protein level of UCP2 in SH-SY5Y could be detected after treatment with AG by standard flow cytometry. (Figure 6).

### Conclusion

These data indicate the first example of a drug-induced upregulation of UCP-2 mRNA. Furthermore, we have found that, as a consequence of this, the level of UCP-2 protein increases and mitochondrial activity and heat flow increase.
This serves as a foundation of an invention related to the drug-induced increase of metabolic efficiency, increase in energy expenditure, and increase thermogenesis by genetic or transcriptional upregulation of UCP-2 in adipose tissue. Drugs that increase energy expenditure are useful in the treatment of obesity, non-insulin dependent diabetes, as well as the metabolic syndrome.

## Claims

1. A method for identifying an agent useful in the treatment or prophylaxis of obesity, metabolic syndrome or non-insulin dependent diabetes mellitus, said method comprising determining whether the said agent up-regulates expression of uncoupling protein 2 (UCP-2).

2. The method according to claim 1 comprising the steps of:
(a) contacting cells expressing UCP-2 with a candidate agent;
(b) cultivating the cells; and
(c) determining whether the said agent up-regulates expression of UCP-2, such upregulation being indicative of an agent useful for treating obesity, metabolic syndrome or non-insulin dependent diabetes mellitus.

3. The method according to claims 1 or 2, wherein a candidate agent is selected that causes an increased level of UCP-2 transcription activity in adipose cells as compared to skeletal muscle cells.

## Patentansprüche

1. Ein Verfahren zum Identifizieren eines Mittels, das zur Behandlung oder Prophylaxe von Fettsucht, Metabolischem Syndrom oder nicht insulinabhängiger Diabetes mellitus nützlich ist, wobei das Verfahren die Bestimmung, ob das Mittel die Expression des Uncouplingproteins 2 (UCP-2) nach oben reguliert, umfasst.

2. Das Verfahren gemäß Anspruch 1, welches die folgenden Schritte umfasst:
(a) Inkontaktbringen von Zellen, die UCP-2 exprimieren, mit einem potentiellen Mittel;
(b) Kultivieren der Zellen; und
(c) Bestimmen, ob das Mittel die Expression von UCP-2 nach oben reguliert, wobei ein solches nach oben Regulieren ein Anzeichen dafür ist, dass das Mittel zur Behandlung von Fettsucht, Metabolischem Syndrom oder nicht insulinabhängiger Diabetes mellitus nützlich ist.

3. Das Verfahren gemäß Anspruch 1 oder 2, in welchem ein potentielles Mittel ausgewählt wird, das ein erhöhtes Niveau der UCP-2-Transkriptionsaktivität in Fettzellen im Vergleich zu Skelettmuskelzellen bewirkt.

## Revendications

1. Méthode pour identifier un agent utile dans le traitement ou la prophylaxie de l'obésité, du syndrome métabolique ou du diabète sucré non insulinodépendant, ladite méthode comprenant le fait de déterminer si ledit agent stimule l'expression de la protéine 2 découplante (UCP-2).

2. Méthode selon la revendication 1, comprenant les étapes suivantes :
(a) mettre les cellules exprimant UCP-2 en contact avec un agent candidat ;
(b) cultiver les cellules ; et
(c) déterminer si ledit agent stimule l'expression de UCP-2, laquelle stimulation étant un indicateur d'un agent utile pour traiter l'obésité, le syndrome métabolique ou le diabète sucré non insulinodépendant.

3. Méthode selon la revendication 1 ou 2, dans laquelle un agent candidat est sélectionné lorsqu'il entraîne une augmentation du niveau de l'activité de transcription de UCP-2 dans les cellules adipeuses en comparaison avec les cellules des muscles squelettiques.
